Europäisches Patentamt

European Patent Office

Office européen des brevets

(11) Publication number: **0 320 308 B1**

# EUROPEAN PATENT SPECIFICATION

(45) Date of publication of patent specification: **03.11.93**

(51) Int. Cl.[5]: **C12Q 1/68**, C12Q 1/00,
//C07H21/00

(21) Application number: **88311741.8**

(22) Date of filing: **12.12.88**

(54) **Method for detecting a target nucleic acid sequence.**

(30) Priority: **11.12.87 US 131936**

(43) Date of publication of application:
**14.06.89 Bulletin 89/24**

(45) Publication of the grant of the patent:
**03.11.93 Bulletin 93/44**

(84) Designated Contracting States:
**AT BE CH DE ES FR GB GR IT LI LU NL SE**

(56) References cited:
EP-A- 0 130 515      EP-A- 0 185 494
EP-A- 0 236 069      EP-A- 0 246 864
EP-A- 0 324 616      EP-A- 0 336 731

JOURNAL OF BIOCHEMISTRY. vol. 100, 1986,
TOKYO JP pages 123 - 131; M.Takhashi et al.:
"Thermophilic HB8 DNA ligase: Effects of
polyethylene glycols and polyamines on
blunt-end ligation of DNA"

PROCEEDINGS OF THE NATIONAL ACADEMY
OF SCIENCES OF USA. vol. 80, October 1983,
WASHINGTON US pages 5852 - 5856;
S.B.Zimmerman et al.: "Macromolecular
crowding allows blunt-end ligation by DNA
ligases from rat liver or Escherichia coli"

SCIENCE vol. 241, 26 August 1988, WASH-
INGTON US pages 1077 - 1080; U.Landegren
et al.: "A ligase-mediated gene detection
technique"

(73) Proprietor: **ABBOTT LABORATORIES**
**One Abbott Park Road**
**Abbott Park, Illinois 60064-3500(US)**

(72) Inventor: **Backman, Keith C.**
**200 Carlisle Road**
**Bedford, MA 01730(US)**
Inventor: **Wang, Chang-ning J.**
**14 Ansie Road**
**Chelmsford, MA 01824(US)**

(74) Representative: **Deans, Michael John Percy**
**Lloyd Wise, Tregear & CO.**
**Norman House**
**105-109 Strand**
**London WC2R OAE (GB)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

**Description**

This invention relates to the detection of nucleic acid.

Nucleic acid hybridisation has been proposed to detect the presence of a particular nucleic acid in a sample. For example, Falkow U.S. Patent No. 4,358,535 discloses a hybridisation assay in which single-stranded DNA is attached to a filter labeled, single-stranded sample DNA is contacted with the filter; and hybridization between sample DNA and the labeled, hybridised probe is detected on the filter.

Whiteley et al. EP 185494 discloses detecting a target nucleic acid sequence that has a diagnostic portion, by treating the sample with a probe complementary (under low stringency conditions) to the diagnostic portion and then treating the sample with a probe complementary (under high stringency conditions) to a contiguous sequence. The diagnostic and contiguous probes are covalently attached, and the attached probes are detected after unattached probes are removed.

EP-A-246 864 describes a method for discriminating a specific base sequence from a variant base sequence in target DNA. The method involves hybridisation of two probes to adjacent or near adjacent segments on the target strand. After filling in the gap (if necessary) the two probes are ligated and the formation of ligated probes is detected. If the end of a probe to be ligated is mismatched with respect to target, the two probes will not be ligated and discrimination is thus achieved.

EP-A-0 236 069 and Mullis US Patent Nos: 4,683,202 and 4,683,195 disclose a process for amplifying a nucleic acid sequence a process for amplifying a nucleic acid sequence by treating complementary nucleic acid strands with primers and extending the primers using DNA polymerase to form a template for synthesizing the desired nucleic acid. The '195 patent features detecting DNA that has been amplified by that process.

We have discovered a method for detecting the presence and abundance of a target nucleic acid sequence in a sample. The method involves rapid cyclic template-dependent reorganization of an excess of probe sequences at a geometric rate, thereby rapidly increasing the availability of the sequence being detected and ultimately increasing the sensitivity of the assay. The use of this method is particularly advantageous when the target sequence is present in low levels, or when it is an extremely minor component in a sample containing other nucleic acid sequences. The process can be readily adapted to automation making it particularly attractive for use in diagnostic kits.

The invention generally features, in one aspect thereof, detecting a target nucleic acid sequence in a sample using a stoichiometric excess of at least four single stranded nucleic acid probes. For convenience, the first and second probes will be called primary probes, and the third and fourth probes will be called secondary probes. The probes have the following characteristics. The first probe is capable of hybridizing to a first segment of a strand of the target nucleic acid sequence, and the second probe is capable of hybridizing to a second segment of the same strand of the target nucleic acid sequence. The first and second probes are selected to enable joining of the 3′ end of the first probe to the 5′ end of the second probe, when the two probes are hybridized to the target sequence, --i.e., the 5′ end of the first segment of the target sequence strand is positioned relative to the 3′ end of the second segment of that strand to enable joining of the probes. The first probe is also hybridizable to the third probe, and the second probe is hybridizable to the fourth probe.

The assay works as follows in a preferred procedure:

Sample DNA is provided as single-stranded DNA, including two complementary target strands (a primary target strand and a secondary target strand) if the target is double stranded. The four probes are introduced to the sample DNA as four single strands so that the two primary probes hybridize to the primary target strand, and (if the target is double-stranded) the two secondary probes hybridize to the secondary target strand. Next, the primary probes are ligated, forming a primary synthetically fused probe sequence, and (for double-stranded targets) secondary probes are fused forming a secondary synthetically fused probe sequence. The DNA is denatured, in effect doubling the target population in the sample. As the cycle of hybridization, ligation and denaturation is repeated, the population of reorganized detectable fused probes increases at a geometric rate. Where the target is single-stranded, the secondary probes lack a target strand until the second cycle, at which point the primary synthetically fused probe sequence forms a template for the two secondary probes, and the assay proceeds as described above. The technique enables reorganization of the probe sequence, to form the fused probe sequence(s) being detected, at a geometric rate in accordance with the principles described below. Rapid reorganization provides excellent sensitivity, using a simple protocol. Preferably, the cycle is repeated 20-50 times.

It is also preferred that the 5′ end of the first section of the primary target strand abuts (is contiguous with), and is joined by a phosphate bond to, the 3′ end of the second section of the primary strand target, without any intervening sequences, to provide efficient ligation, particularly enzymatic ligation. DNA is the preferred nucleic acid, both for the probes and for the target. The preferred method of separating complementary sequences is by heat denaturation, i.e., melting. Preferably the probes are 10-200 bases long. Additional (fifth, sixth, etc.) probes can be used which hybridize adjacent to the other probes and can be joined to those probes in the same way. However, four probes are sufficient and preferred.

The above described method can be used with sensitive detection systems, particularly systems involving a combination of labeling entities on two different probes. For example, the labeling entity on one probe can be a specific binding partner for an insoluble phase (e.g. biotin for an avidin-functionalized insoluble phase), and the labeling entity on the other probe can be a chromophore or fluorophore. After the insoluble phase has been exposed to the sample and washed, the presence of chromophore or fluorophore on that phase indicates the presence of synthetically fused probe, and thereby indicates the presence of target in the sample.

The invention also features, in an alternative aspect thereof, a kit for performing the assay, including the probes, the ligase, and means to separately contain the probes and the ligase. Apparatus for performing the method includes means to hold a mixture comprising the target sequence, probes and ligase, and means to cycle the temperature of the mixture from a denaturing temperature to a temperature allowing hybridization of the probes to the target. Preferably, the temperature is cycled automatically.

Other features and advantages can be apparent from the following description of the preferred embodiments . In the drawings:

Fig. 1 is a diagrammatic representation of steps in a hybridization assay ; and

Fig. 2 is a graph depicting formation of reorganized probes being detected as a function of cycle number.

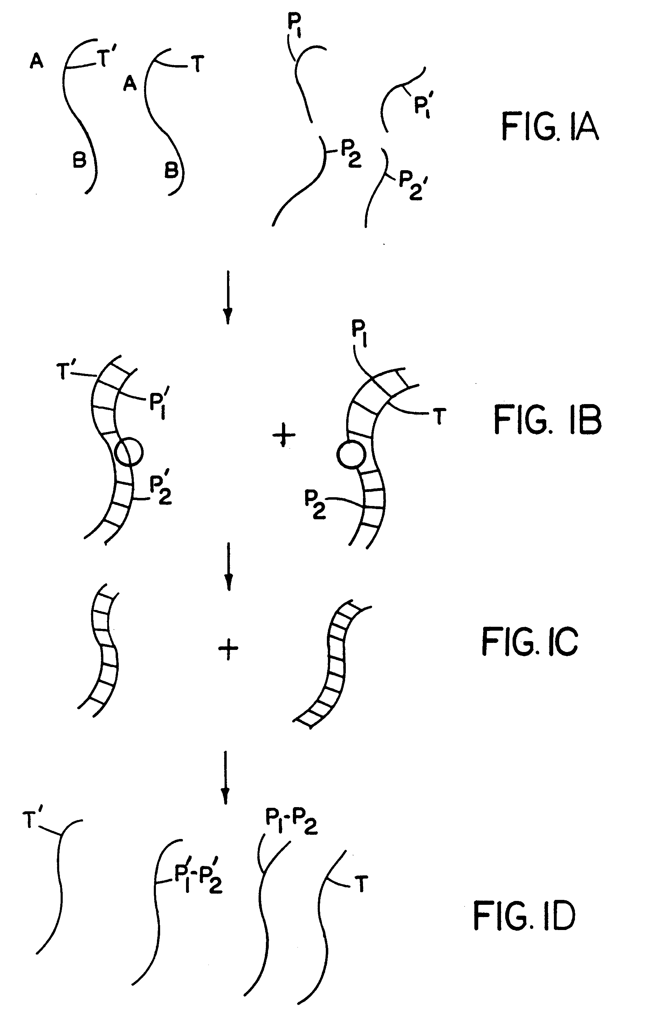

The invention is illustrated by Fig. 1, which depicts steps in a hybridization assay for detecting a nucleotide sequence present in low concentrations.

Those skilled in the field will recognize that there are numerous ways to perform various steps in the method. Generally, the steps can be performed using well-known techniques such as those described in Maniatis et al., Molecular Cloning, Cold Spring Harbor Laboratory (1982). For example, double-stranded DNA can be rendered single-stranded by heat denaturation ("melting") at 80°C - 105°C for 1-5 minutes. Alternatively, enzymatic strand separation can be used. Probes or sub-segments can be synthesized using standard techniques for synthesizing oligonucleotides, or by digesting naturally occurring DNA and isolating fragments. Hybridization conditions will depend on the length and degree of homology of the fragments involved. Generally, the technique and conditions described by Wetmar et al. J. Mol. Biol. 31:349-370 (1968) can be used. Appropriate conditions and techniques for using nucleotide ligases are well known and are supplied by the manufacturer.

Certain features of this system, while not essential, are preferred. In particular, only the 5′ ends that participate in template-dependent joining should be phosphorylated by standard techniques, if they are not already phosphorylated, so as to suppress joining involving other 5′ ends. The lengths and sequences of probes are selected so that, should an incorrect joining of two probes occur (i.e. should two probes join in a manner not represented by a linear sequence on the intended target) those incorrectly joined probes will not serve as a template for the joining of their complementary probes, because the ends of the complementary probes will not be adjacent to each other on a proper manner for enzymatic ligation. Preferably, the probes are between 10 and 200 bases long.

Preferred ligases are those that do not tend to catalyze template independent joining of the probes under at least one set of reaction conditions which is otherwise suitable for the procedure. For example, satisfactory results are achieved with E. coli DNA ligase (available from U.S. Biochemical) or T. thermophilus DNA ligase in the absence of high concentrations of volume excluding solutes, or with T4 DNA ligase on the presence of about 5.0 mM ATP. See, Zimmerman et al., Proc. Nat'l. Acad. Sci. 80, 5852 (1983); Takahashi, M., Uchida,T. J. Biochem. 100, 123 (1986); and Ferreti et al., Nuc. Acids Res. 9, 3695 (1981).

It is also preferred that the ligase enzyme not be denatured by the step intended to dissociate duplex DNA into its constituent strands. Where denaturation is accomplished by increasing the temperature, a thermo-stable ligase is preferable. The benefits of such an enzyme include decreased reagent cost, decreased operating complexity, reduction of amount of undesirable components added (the enzymes are often stored in buffers containing glycerol), and potentially greater shelf life for the reagents. The preferred thermostable ligase is ligase from Thermus thermophilus (e.g. ATCC 27634) purified by the general technique of Takahashi et al., J. Biol. Chem. 259, 10041 (1983).

Fig. 1 shows a hybridization assay detecting a double-stranded target DNA sequence, represented by T-T′ . The target sequence is present in a sample containing many unrelated DNA sequences.

The assay features a kit containing two complementary pairs of probes, represented by $P_1$-$P_1'$ and $P_2$-$P_2'$, in a standard solution. These probes are selected to be complementary to various portions of the target sequence. Specifically, $P_1$ is complementary to segment A of strand T; $P_2$ is complementary to segment B of strand T; $P_1'$ is complementary to segment A of strand T'; and $P_2'$ is complementary to segment B of strand T'. The probes are selected to be long enough to provide selective hybridization, and to generate a fusion sequence that is readily distinguished from other sample components. We have found that probes of 10-200 bases are satisfactory. Most preferably, the probes are between 12 and 50 bases. The probes are provided in large excess to drive the reactions described below. For example, the probe concentration preferably is between about $10^{12}$ and $10^{14}$ molecules per 50 $\mu$L reaction volume.

One cycle of the method is illustrated by Figs. 1A-1D. First (Fig. 1A), the sample DNA is denatured. Then hybridization is permitted (Fig. 1B). If T is present in the sample, there is a relatively high likelihood that T will encounter $P_1$ and $P_2$, and form the species indicated in Fig. 1B. Similarly, T' will encounter $P_1'$ and $P_2'$.

The next step in the cycle is addition of a ligase that will ligate the adjacent probe ends (Fig. 1C), but generally will not ligate blunt ends of DNA in the sample. After ligation, the sample is subjected to denaturing conditions (Fig. 1D), yielding the fused probes $P_1$-$P_2$ and $P_1'$-$P_2'$. From that point, the sample is ready for a new cycle of hybridization-ligation-denaturation.

As will be seen from this example of one cycle, the sample increases from one double-stranded template $T_1$-$T_1'$ at the beginning of the cycle to two double stranded templates. Assuming ideal efficiency in the next cycle, each of these two synthetic double-stranded templates, as well as the original target, will yield two double-stranded templates. Table 1 shows this progression for n cycles, where X is the number of T-$T_1$ pairs before cycle 1.

## Table 1

| No. of Cycles | No. of $P_1$-$P_2$ | No. of $P_1'$-$P_2'$ |
|---|---|---|
| 1 | $1 \cdot X$ | $1 \cdot X$ |
| 2 | $3 \cdot X$ | $3 \cdot X$ |
| 3 | $7 \cdot X$ | $7 \cdot X$ |
| 4 | $15 \cdot X$ | $15 \cdot X$ |
| . | . | . |
| . | . | . |
| . | . | . |
| n | $(2^n-1)X$ | $(2^n-1)X$ |

Since the species $P_1$-$P_2$ (and, if desired $P_1'$-$P_2'$) is detectable, repeated cycles improve detection sensitivity, up to a point. For each cycle, there is a very small but finite chance of forming $P_1$-$P_2$ or $P_1'$-$P_2'$ by blunt end ligation in the absence of T or T'. Once this event occurs, the ligated species is indistinguishable from the presence at the outset of T or T'. Limiting the number of cycles reduces the opportunity for such a false positive reading. Also, at some point the unfused probes are depleted to a level that cannot drive the desired reaction, and there is less chance that fused probes will hybridize with unfused probes (as opposed to the unproductive hybridization of two fused probes).

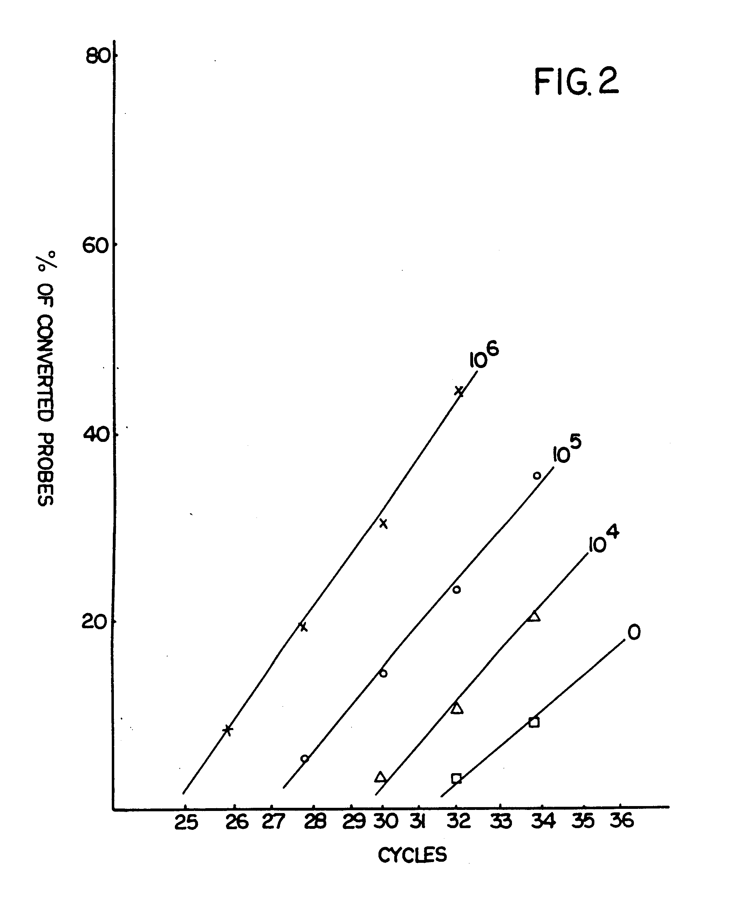

Fig. 2 shows curves depicting the number of detectable fused probes present in the mixture as a function of the number of cycles. Depending on X (the number of target probes originally present), the number of reorganized fused probes will increase geometrically according to the above equation, up to some level at which the rate of increase slows dramatically. By plotting this relationship against standards, and determining how many cycles are required to reach a given level, it is possible to determine the quantity of target present initially.

C. Example 1

Four deoxyribonucleotide oligomers were prepared by standard methods. The oligomers had the following sequences:

$P_1$ = 5' GGGGATCCTCTAGAGTCGACCTGCA 3'

$P_2$ = 5′ AATTCGAGCTCGGTACCC 3′
$P_1'$ = 5′ GGTCGACTCTAGAGGATCCCC 3′
$P_2'$ = 5′ GGGTACCGAGCTCG 3′

$P_1$ and $P_2$ are abutting sequences on one strand of the polylinker region of the plasmid pUC18, and plasmids $P_1'$ and $P_2'$ are abutting sequences on the complementary strand.

Primers $P_1'$ and $P_2$ were treated with polynucleotide kinase and ATP to render their 5′ ends phosphorylated. Primer $P_1$ was radioactively labeled at its 3′ end by treatment with terminal transferase and $\alpha$-$^{32}$P-dCTP.

D. Example 2

Samples were prepared which contained 30mM TrisCl pH8.0, 100 mM NaCl, 1.2mM EDTA, 4.0mM $MgCl_2$, 1.0mM dithiothreitol, 50$\mu$/ml Bovine Serum Albumin, 20$\mu$g/ml of Hela DNA plus 20$\mu$g/ml nonspecific oligonucleotide DNA (e.g., the following 20 mer: 5′-ATCGATACATCAGGAATATT-3′), 1$\mu$g/ml of each of the probes of Example 1 and various amounts of pUC18 plasmid DNA linearized at the EcoRI cleavage site. 50$\mu$l aliquots of these samples were subjected to the following steps:

(a) heat to 100°C for 1 minute to denature the DNA
(b) incubate at 37°C for 1 minute to allow DNA renaturation
(c) add 50 units E. coli DNA ligase (using units defined by the manufacturer, United States Biochemical Corporation)
(d) incubate at 37°C for 1 minute to allow joining of appropriately juxtaposed probes

Steps (a) through (d) were repeated between 20 and 50 times. Aliquots were removed, treated to destroy residual ligase activity, and saved. The saved aliquots were analyzed by polyacrylamide gel electrophoresis and autoradiography. The time of appearance (in number of cycles) of detectable quantities of joined material strongly correlates with the number of target molecules initially present in the reaction.

Other embodiments are feasible.

For example, RNA can be used as well as DNA.

In the examples, Hela DNA and a nonspecific oligonucleotide were included to protect the probe from degradation by nucleases that might be present in the sample. However, these are not essential.

**Claims**

1. A method of detecting target nucleic acid in a sample comprising the steps of:
    (a) providing nucleic acid of the sample as single-stranded nucleic acid;
    (b) providing in the sample a stoichiometric excess of at least four nucleic acid probes, wherein: i) the first and second of said probes are primary probes, and the third and fourth of said probes are secondary nucleic acid probes; ii) the first probe is a single strand capable of hybridizing to a first segment of a primary strand of the target nucleic acid; iii) the second probe is a single strand capable of hybridizing to a second segment of said primary strand of the target nucleic acid sequence; iv) the 5' end of the first segment of said primary strand of the target is positioned relative to the 3' end of the second segment of said primary strand of the target to enable joining of the 3' end of the first probe to the 5' end of the second probe, when said probes are hybridized to said primary strand of said target nucleic acid; v) the third probe is capable of hybridizing to the first probe; and vi) the fourth probe is capable of hybridizing to the second probe; and
    (c) repeatedly performing the following cycle:
        i) hybridizing said probes with nucleic acid in said sample;
        ii) ligating hybridized probes to form reorganized fused probe sequences; and
        iii) denaturing DNA in said sample; and
    (d) detecting the reorganized fused probe sequences;
    whereby with successive cycles the quantity of reorganized fused primary and fused secondary probes is increased.

2. A method according to Claim 1, wherein the 5' end of the first segment of said primary strand of the target sequence abuts, and is joined by a covalent bond to, the 3' end of the second segment of said primary strand of the target sequence, without intervening bases.

3. A method according to Claims 1 or 2, wherein the probes are joined by a ligating enzyme.

**4.** A method according to Claim 3, wherein the probes are joined by a ligase, preferably a bacterial ligase.

**5.** A method according to Claim 4, wherein the ligase is Escherichia coli DNA ligase or Thermus thermophilus DNA ligase.

**6.** A method according to Claim 3, wherein the probes are joined by a thermostable ligating enzyme.

**7.** A method according to any preceding claim, wherein the nucleic acid probes are DNA.

**8.** A method according to any preceding claim, wherein the target nucleic acid sequence is DNA.

**9.** A method according to any preceding claim, wherein the fused nucleic acid is separated from the target sequence by heat denaturation.

**10.** A method according to any preceding claim, wherein said cycle is repeated at least twice, preferably between 20 and 50 times.

**11.** A method according to any preceding claim, wherein the 5' end of the second probe but not of the first probe is phosphorylated.

**12.** A method according to any preceding claim, wherein the target sequence is double-stranded before step (a).

**13.** A method according to any preceding claim, wherein at least one of said probes is labelled with a labelling entity.

**14.** A method according to Claim 13, wherein both of said primary probes are labelled with a labelling entity.

**15.** A method according to Claims 13 or 14, wherein both of said secondary probes are labelled with a labelling entity.

**16.** A method according to any of Claims 13, 14 or 15, wherein the or at least one said labelling entity comprises a chromophore or flurophore.

**17.** A method according to any of Claims 13, 14 or 15, wherein the or at least one said labelling entity comprises a specific binding partner for an insoluble phase.

**18.** A composition for use in a method of detecting target nucleic acid in a sample, said composition comprising a plurality of at least four nucleic acid probes, wherein: i) the first and second of said probes are primary probes, and the third and fourth of said probes are secondary probes; ii) the first probe is a single strand capable of hybridizing to a first segment of a primary strand of the target nucleic acid; iii) the second probe is a single strand capable of hybridizing to a second segment of said primary strand of the target nucleic acid sequence, the 5' end of the first segment of said primary strand of the target being positioned relative to the 3' end of the second segment of said primary strand of the target to enable joining of the first probe to the second probe when said probes are hybridized to said primary strand of said target nucleic acid; iv) the third probe is capable of hybridizing to the first probe; and v) the fourth probe is capable of hybridizing to the second probe such that, when the first and second probes are joined, the third and fourth probes are positioned relative to one another to enable joining of the third probe to the fourth probe.

**19.** A kit for performing an assay in accordance with any of claims 1-17 comprising:
   a) the probe composition of Claim 18;
   b) a nucleic acid ligase; and
   c) means adapted to contain said probe composition and means adapted to contain said nucleic acid ligase.

**Patentansprüche**

1. Verfahren zur Detektion einer Ziel-Nucleinsäure in einer Probe, umfassend die Stufen:
   (a) Bereitstellen der Nucleinsäure der Probe als einsträngige Nucleinsäure;
   (b) Bereitstellen in der Probe einen stöchiometrischen Überschuß von mindestens vier Nucleinsäure-Sonden, worin: i) die erste und zweite der genannten Sonden primäre Sonden und die dritte und vierte der genannten Sonden sekundäre Nucleinsäure-Sonden darstellen; ii) die erste Sonde ein Einzelstrang darstellt, der in der Lage ist, mit einem ersten Segment eines primären Stranges der Ziel-Nucleinsäure zu hybridisieren; iii) die zweite Sonde einen Einzelstrang darstellt, der in der Lage ist, mit einem zweiten Segment des genannten primären Stranges der Ziel-Nucleinsäure zu hybridisieren; iv) das 5'-Ende des ersten Segments des genannten primären Stranges des Ziels relativ zu dem 3'-Ende des zweiten Segments des genannten primären Stranges des Ziels angeordnet ist, um eine Verknüpfung des 3'-Endes der ersten Sonde mit dem 5'-Ende der zweiten Sonde zu ermöglichen, wenn die genannten Sonden mit dem genannten primären Strang der genannten Ziel-Nucleinsäure hybridisiert werden; v) die dritte Sonde in der Lage ist, mit der ersten Sonde zu hybridisieren; und vi) die vierte Sonde in der Lage ist, mit der zweiten Sonde zu hybridisieren; und
   (c) wiederholtes Durchführen des folgenden Kreislaufs:
   i) Hybridisieren der genannten Sonden mit Nucleinsäure in der genannten Probe;
   ii) Ligasieren der hybridisierten Sonden unter Bildung von reorganisierten fusionierten Sonden-Sequenzen; und
   iii) Denaturieren von DNA in der genannten Probe; und
   (d) Detektieren der reorganisierten fusionierten Sondensequenzen;
   wobei durch die aufeinanderfolgenden Kreisläufe die Menge der reorganisierten fusionierten primären und der fusionierten sekundären Sonde vergrößert wird.

2. Verfahren nach Anspruch 1, bei dem das 5'-Ende des ersten Segments des genannten primären Stranges der Ziel-Sequenz an das 3'-Ende angrenzt und über eine kovalente Bindung mit ihm ohne eine dazwischenliegende Base verbunden wird.

3. Verfahren nach den Ansprüchen 1 oder 2, bei dem die Sonden durch ein Ligasierungsenzym verknüpft werden.

4. Verfahren nach Anspruch 3, bei dem die Sonden durch eine Ligase, vorzugsweise eine bakterielle Ligase, verknüpft werden.

5. Verfahren nach Anspruch 4, bei dem die Ligase Escherichia coli DNA-Ligase oder Thermus thermophilus DNA-Ligase darstellt.

6. Verfahren nach Anspruch 3, bei dem die Sonden durch ein wärmestabiles Ligasierungsenzym verknüpft werden.

7. Verfahren nach irgendeinem vorausgegangenen Anspruch, bei dem die Nucleinsäure-Sonden DNA darstellen.

8. Verfahren nach irgendeinem vorausgegangenen Anspruch, bei dem die Ziel-Nucleinsäuresequenz DNA darstellt.

9. Verfahren nach irgendeinem vorausgegangenen Anspruch, bei dem die fusionierte Nucleinsäure durch eine Hitzedenaturierung von der Ziel-Sequenz getrennt wird.

10. Verfahren nach irgendeinem vorausgegangenen Anspruch, bei dem der genannte Kreislauf mindestens zweimal, vorzugsweise zwischen 20 und 50 mal, wiederholt wird.

11. Verfahren nach irgendeinem vorausgegangenen Anspruch, bei dem das 5'-Ende der zweiten Sonde, jedoch nicht das der ersten Sonde, phosphoryliert wird.

**12.** Verfahren nach irgendeinem vorausgegangenen Anspruch, bei dem die Ziel-Sequenz vor der Stufe (a) doppelsträngig ist.

**13.** Verfahren nach irgendeinem vorausgegangenen Anspruch, bei dem mindestens eine der genannten Sonden mit einer Markierung markiert wird.

**14.** Verfahren nach Anspruch 13, bei dem beide der genannten primären Sonden mit einer Markierung markiert werden.

**15.** Verfahren nach den Ansprüchen 13 oder 14, bei dem beide der genannten sekundären Sonden mit einer Markierung markiert werden.

**16.** Verfahren nach einem der Ansprüche 13, 14 oder 15, bei dem die oder mindestens eine genannte Markierung ein Chromophor oder Fluorophor umfaßt.

**17.** Verfahren nach einem der Ansprüche 13, 14 oder 15, bei dem die oder mindestens eine genannte Markierung einen spezifischen Bindungspartner für eine unlösliche Phase umfaßt.

**18.** Zusammensetzung zur Verwendung bei einem Verfahren zur Detektion von Ziel-Nucleinsäure in einer Probe, wobei die genannte Zusammensetzung umfaßt eine Menge von mindestens vier Nucleinsäure-Sonden, worin i) die erste und zweite der genannten Sonden primäre Sonden darstellen und die dritte und vierte der genannten Sonden sekundäre Sonden darstellen; ii) die erste Sonde ein Einzelstrang darstellt, der in der Lage ist, mit einem ersten Segment eines primären Stranges der Ziel-Nucleinsäure zu hybridisieren; iii) die zweite Sonde einen Einzelstrang darstellt, der in der Lage ist, mit einem zweiten Segment des genannten primären Stranges der Ziel-Nucleinsäuresequenz zu hybridisieren, wobei das 5'-Ende des ersten Segments des genannten primären Stranges des Ziels relativ zu dem 3'-Ende des zweiten Segments des genannten primären Stranges des Ziels angeordnet ist, um eine Verknüpfung der ersten Sonde mit der zweiten Sonde zu ermöglichen, wenn die genannten Sonden mit dem primären Strang der genannten Ziel-Nucleinsäure hybridisiert werden; iv) die dritte Sonde in der Lage ist, mit der ersten Sonde zu hybridisieren; und v) die vierte Sonde in der Lage ist, mit der zweiten Sonde derart zu hybridisieren, daß, wenn die erste und zweite Sonde verknüpft werden, die dritte und vierte Sonde relativ zueinander angeordnet werden, so daß eine Verknüpfung der dritten Sonde mit der vierten Sonde ermöglicht wird.

**19.** Kit zur Durchführung eines Tests nach einem der Ansprüche 1 bis 17, umfassen:
a) die Sonden-Zusammensetzung nach Anspruch 18;
b) eine Nucleinsäure-Ligase; und
c) Mittel, die darauf ausgerichtet sind, die genannte Sonden-Zusammensetzung zu enthalten und Mittel, die darauf ausgerichtet sind, die genannte Nucleinsäure-Ligase zu enthalten.

**Revendications**

**1.** Procédé pour détecter un acide nucléique cible dans un échantillon, comprenant les étapes consistant à :
(a) disposer l'acide nucléique de l'échantillon sous forme d'un acide nucléique monocaténaire ;
(b) disposer dans l'échantillon un excès stoechiométrique d'au moins quatre sondes d'acide nucléique,
dans lequel:i) la première et la seconde desdites sondes sont des sondes primaires, et la troisième et la quatrième desdites sondes sont des sondes d'acide nucléique secondaires;ii) la première sonde est un simple brin capable d'hybridation avec un premier segment d'un brin primaire de l'acide nucléique cible ; iii) la seconde sonde est un simple brin capable d'hybridation avec un second segment dudit brin primaire de la séquence d'acide nucléique cible : iv) l'extrémité 5' du premier segment dudit brin primaire de la cible est positionnée par rapport à l'extrémité 3' du second segment dudit brin primaire de la cible pour permettre une liaison de l'extrémité 3' de la première sonde avec l'extrémité 5' de la seconde sonde, lorsque lesdites sondes sont hybridées audit brin primaire dudit acide nucléique cible ; v) la troisième sonde est capable d'hybridation avec la première sonde ; et vi) la quatrième sonde est capable d'hybridation avec la seconde sonde ; et
(c) réaliser de manière répétée le cycle suivant :

EP 0 320 308 B1

i) hybridation desdites sondes avec l'acide nucléique dans ledit échantillon ;
ii) ligature des sondes hybridées pour former des séquences de sondes fusionnées réorganisées ; et
iii) dénaturer l'ADN dans ledit échantillon ; et
(d) détecter les séquences de sondes fusionnées réorganisées ;
de sorte que, par des cycles successifs, la quantité de sondes primaires fusionnées et de sondes secondaires fusionnées réorganisées est augmentée.

2. Procédé selon la revendication 1, dans lequel l'extrémité 5' du premier segment dudit brin primaire de la séquence cible est contiguë et liée par une liaison covalente à l'extrémité 3' du second segment dudit brin primaire de la séquence cible, sans bases intermédiaires.

3. Procédé selon la revendication 1 ou 2, dans lequel les sondes sont liées par une enzyme de ligature.

4. Procédé selon la revendication 3, dans lequel les sondes sont liées par ligase, de préférence une ligase bactérienne.

5. Procédé selon la revendication 4, dans lequel la ligase et l'ADN ligase de Escherichia coli ou l'ADN ligase de Thermus thermophilus.

6. Procédé selon la revendication 3, dans lequel les sont liées par une enzyme de ligature thermostable.

7. Procédé selon l'une quelconque des revendications précédentes, dans lequel les sondes d'acide nucléique sont de l'ADN.

8. Procédé selon l'une quelconque des revendications précédentes, dans lequel la séquence d'acide nucléique cible est de l'ADN.

9. Procédé selon l'une quelconque des revendications précédentes, dans lequel l'acide nucléique fusionné est séparé de la séquence cible par dénaturation thermique.

10. Procédé selon l'une quelconque des revendications précédentes, dans lequel ledit cycle est répété au moins deux fois, de préférence 20 à 50 fois.

11. Procédé selon l'une quelconque des revendications précédentes, dans lequel l'extrémité 5' de la seconde sonde est phosphorylée mais pas celle de la première sonde.

12. Procédé selon l'une quelconque des revendications précédentes, dans lequel la séquence cible est bicaténaire avant l'étape (a).

13. Procédé selon l'une quelconque des revendications précédentes, dans lequel au moins l'une desdites sondes est marquée par une entité de marquage.

14. Procédé selon la revendication 13, dans lequel les deux sondes primaires sont marquées par une entité de marquage.

15. Procédé selon la revendication 13 ou 14, dans lequel la deux sondes secondaires sont marquées par une entité de marquage.

16. Procédé selon l'une quelconque des revendications 13,14 ou 15, dans lequel l'entité de marquage ou au moins une entité de marquage comprend un chromophore ou un fluorophore.

17. Procédé selon l'une quelconque des revendications 13, 14 ou 15, dans lequel l'entité de marquage ou au moins une entité de marquage comprend un partenaire de liaison spécifique pour une phase insoluble.

18. Composition utilisable dans un procédé pour détecter un acide nucléique cible dans un échantillon, ladite composition comprenant une multiplicité d'au moins quatre sondes d'acide nucléique, dans

9

laquelle : i) la première et la seconde desdites sondes sont des sondes primaires, et la troisième et la quatrième desdites sondes sont des secondes secondaires ; ii) la première sonde est un simple brin capable d'hybridation avec un premier segment d'un brin primaire de l'acide nucléique cible ; iii) la seconde sonde est un simple brin capable d'hybridation avec un second segment dudit brin primaire de la séquence d'acide nucléique cible, l'extrémité 5' du premier segment dudit brin primaire de la cible étant positionnée par rapport à l'extrémité 3' du second segment dudit brin primaire de la cible pour permettre une liaison de la première sonde à la seconde sonde lorsque lesdites sondes sont hybridées audit brin primaire dudit acide nucléique cible ; iv) la troisième sonde est capable d'hybridation avec la première sonde ; et v) la quatrième sonde est capable d'hybridation avec la seconde sonde de sorte que, lorsque les première et seconde sondes sont liées, les troisième et quatrième sondes sont positionnées l'une par rapport à l'autre pour permettre une liaison de la troisième sonde à la quatrième sonde.

19. Trousse pour réaliser une détermination selon l'une quelconque des revendications 1 à 17, comprenant :

a) la composition de sondes de la revendication 18 ;

b) une ligase d'acide nucléique ; et

c) des dispositifs conçus pour contenir ladite composition de sondes et des dispositifs conçus pour contenir ladite ligase d'acide nucléique.

FIG. IA

FIG. IB

FIG. IC

FIG. ID

FIG. 2